(19) Europäisches Patentamt
European Patent Office
Office européen des brevets

(11) Veröffentlichungsnummer: **0 596 284 A2**

# (12) EUROPÄISCHE PATENTANMELDUNG

(21) Anmeldenummer: **93116526.0**

(22) Anmeldetag: **13.10.93**

(51) Int. Cl.5: **A61K 7/48**, A61K 31/22, A61K 47/14, A23L 3/3517

(30) Priorität: **05.11.92 DE 4237367**

(43) Veröffentlichungstag der Anmeldung: **11.05.94 Patentblatt 94/19**

(84) Benannte Vertragsstaaten: **AT BE CH DE DK ES FR GB IT LI NL PT SE**

(71) Anmelder: **Beiersdorf Aktiengesellschaft**
**Unnastrasse 48**
**D-20245 Hamburg(DE)**

(72) Erfinder: **Wolf, Florian, Dr.**
**Husumer Strasse 2**
**D-20251 Hamburg(DE)**
Erfinder: **Kahlert, Michael**
**Walddörfer Strasse 59**
**D-22041 Hamburg(DE)**

(54) **Antimycotische kosmetische und dermatologische Zubereitungen mit einem Gehalt an Fettsäureestern.**

(57) Verwendung eines oder mehrerer Fettsäureester ein- und/oder mehrwertiger Alkohole als antimycotisches Wirkprinzip in kosmetischen oder dermatologischen Zubereitungen.

EP 0 596 284 A2

Die vorliegende Erfindung betrifft antimycotisch wirksame und gegen den Befall mit Mycobionten geschützte Zubereitungen, bevorzugt kosmetischen oder dermatologischen Zubereitungen.

Pilze, auch Fungi [fungus = lat. Pilz], Mycota [mykes = grch. Pilz] oder Mycobionten genannt, zählen zu den Eucaryonten. Eucaryonten sind Lebewesen, deren Zellen (Eucyten) im Gegensatz zu denen der sogenannten Procaryonten (Procyten) über einen durch Kernhülle und Kernmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und natürlich die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z.B. Epidermophytie, Favus, Mikrosporie, Trichophytie), Hefemycosen (z.B. Pityriasis, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose, Skopulariopsidose), Systemmycosen (z.B. Chromomycose, Coccidiomycose, Histoplasmose).

Zu den pathogenen oder fakultativ pathogenen Keimen gehören aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche.

Schließlich können durch Mycobiontenbefall Zubereitungen, insbesondere Nahrungsmittel, aber auch Kosmetika und dergleichen zerstört werden. Der Schutz vor Infektion erstreckt sich daher auch auf die Produkte selbst.

Behandelt werden Dermatomycosen medikamentös oder mit anderen Methoden, beispielsweise mit Lichtbestrahlung. Gängige Medikamente enthalten Salicylsäure, Kresol, 1-Menthol und andere, die alle äußerlich angewandt werden und regelmäßig gute Heilerfolge zeitigen.

Dennoch haben die bisher bekannten antimycetisch wirksamen Mittel den Nachteil daß sie unangenehm riechen und/oder die beeinträchtigte Hautpartie zusätzlich reizen. In schweren Fällen von Dermatomycosen kann durch das Medikament sogar Schmerz ausgelöst werden.

Die Aufgabe der vorliegenden Erfindung war also, ein antimikrobiell wirksames Mittel zur Verfügung zu stellen, welches die Nachteile des Standes der Technik nicht aufweist. Insbesondere sollten Wirkstoffe zugängig gemacht werden, die folgende Bedingungen erfüllen:

1) Die biologischen Vorgänge der Haut dürfen nicht beeinträchtigt werden.
2) Die Wirkstoffe sollen keinen ausgeprägten Eigengeruch besitzen.
3) Sie sollen bei Überdosierung oder sonstiger nicht bestimmungsgemäßer Anwendung unschädlich sein.
4) Sie sollen sich nach wiederholter Anwendung nicht auf der Haut anreichern.
5) Pathogene Mycobionten sollen vernichtet oder wenigstens stark dezimiert werden, die physiologische Mikroflora der Haut im übrigen aber geschont werden.
6) Die wirksamen Prinzipien sollen sich gut in übliche kosmetische oder dermatologische Formulierungen einarbeiten lassen.
7) Die Wirkstoffe sollen die Haut nicht reizen.

8) Zubereitungen, diese Wirkstoffe enthaltend, sollen ebenfalls vor Befall mit Mycobionten geschützt sein.

Es wurde gefunden, und darin liegt die Lösung all dieser Aufgaben, daß

die Verwendung eines oder mehrerer Fettsäureester ein- und/oder mehrwertiger Alkohole als antimycotisches Wirkprinzip in kosmetischen oder dermatologischen Zubereitungen

den Nachteilen des Standes der Technik abhilft.

Es hat sich in erstaunlicher Weise herausgestellt, daß die erfindungsgemäßen Fettsäureester ein- und/oder mehrwertiger Alkohole das Wachstum von Mycobionten verhindern.

Insbesondere sind die erfindungsgemäßen Fettsäureester ein- und/oder mehrwertiger Alkohole befähigt, daß Wachstum von Hefen, insbesondere der Pityrosporum-Arten, namentlich Pityrosporum ovale, zu verhindern.

Es hat sich ferner herausgestellt, daß die erfindungsgemäßen Fettsäureester ein- und/oder mehrwertiger Alkohole die Bildung von seborrhoischen Erscheinungen, insbesondere Kopfschuppen, verhindern sowie bereits vorhandene seborrhoische Erscheinungen, insbesondere Kopfschuppen, zu beseitigen.

Erfindungsgemäß ist somit auch ein Verfahren zur Bekämpfung seborrhoischer Erscheinungen, insbesondere Kopfschuppen, sowie die Prophylaxe gegen seborrhoische Erscheinungen, insbesondere Kopfschuppen.

Schließlich hat sich herausgestellt, daß die erfindungsgemäßen Fettsäureester ein- und/oder mehrwertiger Alkohole den Verderb organischer Substanz, insbesondere kosmetischer und dermatologischer Zubereitungen, durch den Befall mit Mycobionten verhindern können, wenn sie diesen Zubereitungen zugesetzt werden.

Erfindungsgemäß sind somit auch ein Verfahren zur Bekämpfung von Mycobionten, dadurch gekennzeichnet, daß ein oder mehrere Fettsäureester ein- und/oder mehrwertige Alkohole, gegebenenfalls in einem geeigneten kosmetischen oder dermatologischen Träger, mit dem durch Mycobionten kontaminierten Bereich in Kontakt gebracht werden, sowie ein Verfahren zum Schutze organischer Produkte vor dem Befall mit Mycobionten, dadurch gekennzeichnet, daß diesen organischen Produkten ein oder mehrere Fettsäureester ein- und/oder mehrwertiger Alkohole in wirksamer Menge zugegeben werden.

Fettsäurester im Sinne der vorliegenden Erfindung sind Ester der allgemeinen Formel

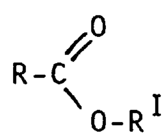 ,

welche formal Verestererungsprodukte einer Fettsäure der allgemeinen Formel

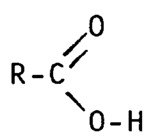

sowie eines ein- oder mehrwertigen Alkohols der allgemeinen Formel

$R^I$-O-H

darstellen. R stellt dabei einen verzweigten oder unverzweigten Alkylrest einer Kettenlänge von $C_{1-25}$ dar. $R^I$ stellt einen verzweigten oder unverzweigten $C_{1-25}$-Alkylrest dar, wobei auch ein oder mehrere Wasserstoffatome dieses Alkylrestes durch eine oder mehrere $OR^{II}$- Reste substituiert sein können, wobei dieser $R^{II}$-Rest oder diese -$R^{II}$-Reste unabhängig voneinander aus der Gruppe H, verzweigtes oder unverzweigtes $C_{1-25}$-Alkyl und/oder verzweigtes oder unverzweigtes $C_{1-25}$-Acyl gewählt werden können.

Vorteilhaft können diese Reste so gewählt werden, daß R dabei einen verzweigten oder unverzweigten Alkylrest einer Kettenlänge von $C_{6-18}$ darstellt, $R^I$ aus der Gruppe verzweigtes oder unverzweigtes $C_{1-10}$-Alkyl-, wobei auch ein oder mehrere Wasserstoffatome dieses Alkylrests durch eine oder mehrere $OR^{II}$-Reste substituiert sein können, wobei dieser $R^{II}$-Rest oder diese -$R^{II}$-Reste unabhängig voneinander aus der Gruppe H, verzweigtes oder unverzweigtes $C_{6-18}$-Alkyl oder $C_{6-18}$-Acyl gewählt werden können. Besonders vorteilhaft stellt R dabei einen Alkylrest einer Kettenlänge von $C_{6-18}$ dar, $R^I$ wird günstig gewählt aus

der Gruppe
Hexyl-, Isopropyl-, sowie aus der Gruppe

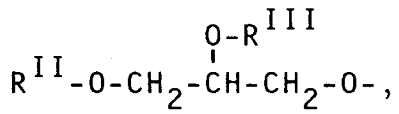

wobei $R^{II}$ und $R^{III}$ unabhängig voneinander aus der Gruppe H-, verzweigtes oder unverzweigtes $C_{6-18}$-Alkyl- oder $C_{6-18}$-Acyl- gewählt werden können.

Bevorzugt ist insbesondere, die Fettsäureester zu wählen aus der Gruppe Hexyllaurat, Isopropylstearat, Glycerylmonolaurat, Caprylsäure-/Caprinsäuretriglycerid.

Erfindungsgemäß werden die Fettsäureester ein- und/oder mehrwertiger Alkohole bevorzugt in kosmetischen oder dermatologischen Zusammensetzungen eingesetzt, wobei diese vorteilhaft einen Gehalt von 0,01 - 20,0 Gew.-% , bezogen auf das Gesamtgewicht der Zusammensetzung, enthalten. Besonders vorteilhaft enthalten die Zusammensetzungen 0,02 - 5,0 Gew.-% an den erfindungsgemäßen Fettsäureestern ein- und/oder mehrwertiger Alkohole, ganz besonders vorteilhaft 0,5 - 3,0 Gew.-%, jeweils bezogen auf das Gesamtgewicht der Zusammensetzung.

Die erfindungsgemäßen Fettsäureester ein- und/oder mehrwertiger Alkohole lassen sich ohne Schwierigkeiten in gängige kosmetische oder dermatologische Formulierungen einarbeiten, vorteilhaft in Pumpsprays, Aerosolsprays, Crèmes, Salben, Tinkturen, Lotionen, Nagelpflegeprodukte (z.B. Nagellacke, Nagellackentferner, Nagelbalsame) und dergleichen.

Ganz besonders bevorzugt ist, die erfindungsgemäßen Fettsäureester ein- und/oder mehrwertiger Alkohole in Antischuppen-Shampoos einzuarbeiten.

Erfindungsgemäß ist daher die Verwendung eines oder mehrerer Fettsäureester ein- und/oder mehrwertiger Alkohole als gegen Kopfschuppen wirksames Prinzip in kosmetischen oder dermatologischen Zubereitungen.

Erfindungsgemäß ist ferner ein Verfahren zur Bekämpfung von Kopfschuppen, dadurch gekennzeichnet, daß ein oder mehrere Fettsäureester ein- und/oder mehrwertige Alkohole gegebenenfalls in einem geeigneten kosmetischen oder dermatologischen Träger, mit dem seborrhoischen Bereich in Kontakt gebracht werden.

Es ist auch möglich und gegebenenfalls vorteilhaft, die erfindungsgemäßen Fettsäureester ein- und/oder mehrwertiger Alkohole mit anderen Wirkstoffen zu kombinieren, beispielsweise mit anderen antimikrobiell wirksamen Stoffen.

Es ist vorteilhaft, die erfindungsgemäßen Zusammensetzungen abzupuffern. Vorteilhaft ist ein pH-Bereich von 3,5 - 9,0. Besonders günstig ist es, den pH-Wert in einem Bereich von 4,0 - 6,0 zu wählen.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:

- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Emulsionen gemäß der Erfindung z.B. in Form einer Sonnenschutzcreme, einer Sonnenschutzlotion oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrigalkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte.

Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bevorzugt können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen.

Kosmetische Zubereitungen gemäß der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel. Die kosmetischen Zubereitungen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Dusch- oder Badezubereitung darstellen, enthalten vorzugsweise mindestens eine anionische, nichtionische oder amphotere oberflächenaktive Substanz oder Gemische daraus, mindestens einen erfindungsgemäßen Fettsäureester im wäßrigen Medium und Hilfsmittel, wie sie üblicherweise dafür verwendet werden. Die oberflächenaktive Substanz kann in einer Konzentration zwischen 1 Gew.-% und 50 Gew.-% in dem Shampoonierungsmittel,

bzw. der Wasch-, Dusch- oder Badezubereitung, vorliegen.

Liegt die kosmetische oder dermatologische Zubereitung in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetische oder dermatologische Zubereitung kann auch ein Aerosol mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie mindestens einen erfindungsgemäßen Fettsäureester. Die Menge der verwendeten Polymeren liegt z.B. zwischen 0,1 und 10 Gew.-%, bevorzugt zwischen 0,1 und 3 Gew.-%.

Kosmetische und dermatologische Zubereitungen zur Behandlung und Pflege der Haare, die mindestens einen erfindungsgemäßen Fettsäureester enthalten, können als Emulsionen vorliegen, die vom nichtionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen. Anionische Emulsionen sind vorzugsweise vom Typ einer Seife und enthalten mindestens einen erfindungsgemäßen Fettsäureester.

Kosmetische und dermatologische Zubereitungen zur Behandlung und Pflege der Haare können als Gele vorliegen, die neben mindestens einen Fettsäureester ein- und/oder mehrwertiger Alkohole und dafür üblicherweise verwendeten Lösungsmitteln noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdikkungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge der erfindungsgemäßen Fettsäureester ein- und/oder mehrwertiger Alkohole in einem für die Haare bestimmten Mittel 0,01 Gew.-% bis 10 Gew.%, insbesondere 0,5 Gew.-% bis 5 Gew.-%, bezogen auf das Gesamtgewicht der Zubereitungen.

Anhand der folgenden Versuche soll die erstaunliche antimykotische Wirkung der erfindungsgemäßen Fettsäureester demonstriert werden:

Versuch 1

Testlösungen:

| | |
|---|---|
| TY-Medium | 10 g Trypton + 5 g Hefeextrakt + 10 g NaCl, aufgelöst in 1000 ml $H_2O$, gepuffert auf pH 7,4 , autoklaviert. |
| Testverbindung 1: | Hexyllaurat |
| Testverbindung 2: | Isopropylstearat |
| Testverbindung 3: | Caprylsäure-/Caprinsäure-Triglycerid |
| Testverbindung 4: | Glyceryllaurat |

Pityrosporum ovale von drei Patienten mit Pityrosporum-Follikulitis, Pityriasis versicolor oder seborrhoischem Ekzem wurde in einer Mischung aus 50 % TY-Medium, 30 % Olivenöl und 20 % Tween 80 suspendiert. Die drei verschiedenen Hefestämme wurden in drei parallelen Ansätzen auf einer Selektivagarplatte für pathogene Pilze (Merck AG, Darmstadt) gleichmäßig ausgespatelt. Mit Hilfe steriler Korkbohrer wurden in jede Platte mehrere kreisrunde Vertiefungen gestanzt, in die Vertiefungen mit einem Durchmesser von 5 mm wurden jeweils 25 ul der Testlösungen gegeben. Die Testlösungen stellten 0,5 %-ige, 1,0 %-ige, 2,5 %-ige, 5,0 %-ige und 10,0 %-ige Lösungen der Testsubstanzen in Ethanol dar.

Nach dreistündiger Inkubation bei 37° C wurde das verdunstete Lösungsmittel durch je 25 ul Jojobaöl ersetzt und die Platten anschließend unter Beibehaltung der Wachstumsbedingungen drei Tage inkubiert. Während dieser Zeit diffundierten die Testsubstanzen in den die Vertiefungen umgebenden Nährboden.

Antimycotisch wirksame Testverbindungen zeigten nach dreitägiger Inkubation einen deutlichen konzentrischen Hemmhof um die Vertiefungen. Die Größe dieser Hemmhöfe, wiedergegeben in Tabelle 1, ist bei

vergleichbarer Substanzkonzentration ein Maß für die antimycotische Wirksamkeit der Testverbindungen.

Tabelle 1

| | mittlerer Hemmhof-Durchmesser [mm] | | | |
|---|---|---|---|---|
| Konz.: | 0,5 % | 1,0 % | 5,0 % | 10,0 % |
| Testlösung 1 | 14,0 | 15,5 | 18,0 | 20,7 |
| Testlösung 2 | 10,7 | 14,0 | 18,7 | 20,7 |
| Testlösung 3 | 9,0 | 9,0 | 15,0 | 17,3 |
| Testlösung 4 | 11,0 | 14,0 | 16,5 | 16,5 |

Die nachfolgenden Beispiele sollen die vorliegende Erfindung verdeutlichen, ohne sie einzuschränken.

Beispiel 1

Sonnenschutzcrème

| | Gew.-% |
|---|---|
| Glycerylcocoat + hydriertes Kokosöl + Ceteareth-25 ("Softisan 601") | 35,00 |
| Caprylsäure-/Caprinsäuretriglycerid ("Miglyol 812") | 7,00 |
| Glycerylstearat ("Imwitor 960") | 5,00 |
| Caprylsäurediethylamid ("Repellent 790") | 3,00 |
| Dihydroxyaceton | 0,50 |
| Phenylbenzimidazolsulfonsäure ("Eusolex 232") | 4,00 |
| Konservierungsmittel, Parfum, Stabilisatoren usw. | nach Belieben |
| Wasser | ad 100,00 |

Beispiel 2

Tagescrème

| | Gew.-% |
|---|---|
| Glycerylstearat ("Imwitor") | 15,00 |
| Caprylsäure/Caprinsäure-Triglycerid ("Miglyol 812") | 15,00 |
| Paraffinöl DAB 9 | 5,00 |
| Glycerin-Polyethylenglycolricinoleat ("Cremophor EL") | 2,00 |
| Konservierungsmittel, Parfum, Stabilisatoren usw. | nach Belieben |
| Wasser | ad 100,00 |

Beispiel 3

Kollagencrème

| | Gew.-% |
|---|---|
| Glycerylstearat ("Imwitor") | 9,00 |
| Paraffinöl DAB 9 | 5,00 |
| Isopropylmyristat | 5,00 |
| Cetylalcohol | 1,00 |
| Stearinsäure Caprylsäure/Caprinsäure-Triglycerid | 5,00 |
| ("Miglyol 812") | 5,00 |
| Sorbitlösung 70 % | 5,00 |
| Triethanolamin | 0,90 |
| Kollagen CLR Konservierungsmittel, Parfum, | 5,00 |
| Stabilisatoren usw. | nach Belieben |
| Wasser | ad 100,00 |

Beispiel 4

O/W-Diglycerincrème

| Phase A | |
|---|---|
| | Gew.-% |
| Glycerylstearat ("Imwitor 900") | 9,50 |
| Cetearylalkohol ("Lanette O" | 1,60 |
| Cetearylisethionat ("Cetiol SN") | 4,00 |
| Cetylacetat + Acetylierter Lanolinalkohol ("Acetulan") | 2,40 |
| Calendulaöl | 1,60 |
| Isopropylstearat | 0,75 |
| Glycerinmonolaurat | 0,75 |

| Phase B | |
|---|---|
| Diglycerin | 8,00 |
| Glycerin | 8,00 |
| Carbomer/Polyacrylat ("Carbopol 940") | 0,12 |
| Triethanolamin 98 % | 0,22 |
| Kamillenextrakt | 1,00 |
| Konservierungsmittel, Parfum, Stabilisatoren usw. | nach Belieben |
| Wasser | ad 100,00 |

Die Phasen A und B werden separat auf 60° C erwärmt, unter Rühren zusammengegeben und sodann langsam auf Raumtemperatur abgekühlt.

Beispiel 5

Ölbad, flüssig

| | Gew.-% |
|---|---|
| Oleth-5 ("Eumulgin 0 5") | 10,00 |
| Laurinsäurehexylester ("Cetiol A") | 40,00 |
| Cocosfettsäurediethanolamid ("Comperlan") | 10,00 |
| Caprylsäure-/Caprinsäure-Triglycerid ("Myritol 318") | 10,00 |
| Paraffinöl DAB 9 | 25,00 |
| Parfum | 5,00 |

Beispiel 6

Kräuter-Ölbad

| | Gew.-% |
|---|---|
| Fettalkoholpolyether ("Aethoxal B") | 52,00 |
| Laureth-2 ("Dehydol LS 2" | 10,00 |
| Laurinsäurehexylester ("Cetiol A") | 15,00 |
| Caprylsäure-/Caprinsäure-Triglycerid ("Myritol 318") | 20,00 |
| Rosmarinöl | 3,00 |

Beispiel 7

Antischuppenshampoo klar

| | Gew.-% |
|---|---|
| Allantoin | 0,20 |
| Natrium-alkyl-polyglycolethersulfat ("Genapol LRO flüssig") | 25,00 |
| Natrium-alkyl-polyglycolethersulfat ("Genapol AMG") | 10,00 |
| Caprylsäure-/Caprinsäure-Triglycerid ("Myritol 318") | 10,00 |
| NaCl | 1,10 |
| Konservierungsmittel, Parfum, Stabilisatoren usw. | nach Belieben |
| Wasser | ad 100,00 |

Beispiel 8

Antischuppenshampoo mit Perlglanz

| | Gew.-% |
|---|---|
| Natrium-alkyl-polyglycolethersulfat ("Genapol LRO flüssig") | 40,00 |
| Natrium-alkyl-polyglycolethersulfat ("Genapol PGL") | 4,00 |
| Fettsäure-alkylolamidpolyglycolether ("Genagen CAB") | 10,00 |
| Caprylsäure-/Caprinsäure-Triglycerid ("Myritol 318") | 10,00 |
| NaCl | 0,50 |
| Konservierungsmittel, Parfum, Stabilisatoren usw. | nach Belieben |
| Wasser | ad 100,00 |

Die erfindungsgemäßen Formulierungen gemäß den Beispielen 1 - 8 sind hervorragend gegen Befall von Mycobionten geschützt. Die Antischuppenshampoos gemäß den Beispielen 7 und 8 zeichnen sich durch ausgezeichnete Antischuppenwirkung aus.

**Patentansprüche**

1. Verwendung eines oder mehrerer Fettsäureester ein- und/oder mehrwertiger Alkohole als antimycotisches Wirkprinzip in kosmetischen oder dermatologischen Zubereitungen.

2. Verwendung nach Anspruch 1, dadurch gekennzeichnet, daß die Fettsäurestern gewählt werden aus der Gruppe der Verbindung der allgemeinen Formel

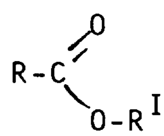
,

wobei
R einen verzweigten oder unverzweigten Alkylrest einer Kettenlänge von $C_{1-25}$ darstellt,
$R^1$ einen verzweigten oder unverzweigten $C_{1-25}$-Alkylrest darstellt, wobei auch ein oder mehrere Wasserstoffatome dieses Alkylrestes durch eine oder mehrere $R^{II-}$ Reste substituiert sein können und wobei dieser $R^{II}$-Rest oder diese -$R^{II}$-Reste unabhängig voneinander aus der Gruppe H, verzweigtes oder unverzweigtes $C_{1-25}$-Alkyl- oder $C_{1-25}$-Acyl- gewählt werden können.

3. Verwendung nach Anspruch 2, dadurch gekennzeichnet, daß die erfindungsgemäßen Fettsäureester ein- oder mehrwertiger Alkohole so gewählt werden, daß
R einen verzweigten oder unverzweigten Alkylrest einer Kettenlänge von $C_{6-18}$ darstellt,
$R^I$ aus der Gruppe verzweigtes oder unverzweigtes $C_{1-10}$-Alkyl gewählt wird, wobei auch ein oder mehrere Wasserstoffatome dieses Alkylrests durch eine oder mehrere $OR^{II}$- Reste substituiert sein können, wobei dieser $R^{II}$-Rest oder diese -$R^{II}$-Reste unabhängig voneinander aus der Gruppe H, verzweigtes oder unverzweigtes $C_{6-18}$-Alkyl- oder $C_{6-18}$-Acyl- gewählt werden können.

4. Verwendung nach Anspruch 2, dadurch gekennzeichnet, daß
R einen Alkylrest einer Kettenlänge von $C_{6-18}$ darstellt,
$R^I$ aus der Gruppe
Hexyl-, Isopropyl- sowie aus der Gruppe

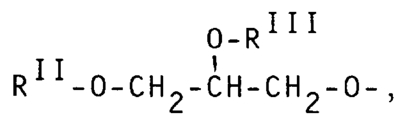

gewählt wird, wobei $R^{II}$ und $R^{III}$ unabhängig voneinander aus der Gruppe H-, verzweigtes oder unverzweigtes $C_{6-18}$-Alkyl- oder $C_{6-18}$-Acyl-gewählt werden.

**5.** Verwendung nach Anspruch 1, dadurch gekennzeichnet, daß die Fettsäureester gewählt werden aus der Gruppe Hexyllaurat, Isopropylstearat, Glycerylmonolaurat, Caprylsäure-/Caprinsäuretriglycerid.

**6.** Verwendung nach Anspruch 1, dadurch gekennzeichnet, daß die Fettsäureester ein- und/oder mehrwertiger Alkohole in kosmetischen oder dermatologischen Zusammensetzungen in einem Gehalt von 0,01 - 20,0 Gew.-%, bevorzugt in einem Gehalt von 0,02 - 5,0 Gew.-% besonders bevorzugt in einem Gehalt von 0,5 - 3,0 Gew.-% vorliegen, jeweils bezogen auf das Gesamtgewicht der Zusammensetzung.

**7.** Verwendung eines oder mehrerer Fettsäureester ein- und/oder mehrwertiger Alkohole als gegen Kopfschuppen wirksames Prinzip in kosmetischen oder dermatologischen Zubereitungen.

**8.** Verfahren zur Bekämpfung von Mycobionten, dadurch gekennzeichnet, daß ein oder mehrere Fettsäureester ein- und/oder mehrwertige Alkohole, gegebenenfalls in einem geeigneten kosmetischen oder dermatologischen Träger, mit dem durch Mycobionten kontaminierten Bereich in Kontakt gebracht werden.

**9.** Verfahren zum Schutze organischer Produkte vor dem Befall mit Mycobionten, dadurch gekennzeichnet, daß diesen organischen Produkten ein oder mehrere Fettsäureester ein- und/oder mehrwertiger Alkohole in wirksamer Menge zugegeben werden.

**10.** Verfahren zur Bekämpfung von Kopfschuppen, dadurch gekennzeichnet, daß ein oder mehrere Fettsäureester ein- und/oder mehrwertige Alkohole, gegebenenfalls in einem geeigneten kosmetischen oder dermatologischen Träger, mit dem seborrhoischen Bereich in Kontakt gebracht werden.